# EUROPEAN PATENT SPECIFICATION

(11) **EP 1 249 242 B1**
(45) Date of publication and mention of the grant of the patent: **27.10.2004**
(21) Application number: 01830246.3
(22) Date of filing: 10.04.2001
(51) Int. Cl.: A61K 35/78

(54) **Natural-Phytotherapic composition based on hydroglyceric extracts for aerosol therapy**
Hydroglycerol-Extrakte enthaltende Phytotherapeutika zur Verwendung als Aerosole
Composition phytothérapeutique à base des extraits hydroglycériques pour administration par aérosol

(43) Date of publication of application: 16.10.2002
(73) Proprietor: Docteur Nature Srl, 41041 Baggiovara (Modena) (IT)
(72) Inventor: Barberini, Stefano, 41041 Casinalbo (MO) (IT)
(74) Representative: Robba, Pierpaolo

(56) References cited:
- DATABASE BIOSIS [Online] BIOSCIENCES INFORMATION SERVICE, PHILADELPHIA, PA, US; BELOMYTTSEVA L A ET AL: "EXPERTISE IN USING PROPOLIS AEROSOL INHALATION IN TREATING PATIENTS WITH OCCUPATIONAL DISEASES OF THE RESPIRATORY ORGANS." retrieved from STN, accession no. PREV197763046672 XP002178377 & GIG TR PROF ZABOL, (1976) (4), 54.,
- FOCHT J ET AL: "Bactericidal effect of propolis in vitro against agents causing upper respiratory tract infections." ARZNEIMITTEL-FORSCHUNG, (1993 AUG) 43 (8) 921-3., XP001019193
- DATABASE WPI Section Ch, Week 199318 Derwent Publications Ltd., London, GB; Class B04, AN 1993-150104 XP002178378 & SU 1 732 999 A (KIEV PHARMACOLOGY TOXICOLOGY), 15 May 1992 (1992-05-15)
- LOGGIA R DELLA ET AL: "Anti-inflammatory activity of some Ginkgo biloba constituents and of their phospholipid-complexes." FITOTERAPIA, vol. 67, no. 3, 1996, pages 257-264, XP001021842 ISSN: 0367-326X
- DECLUME C: "ANTI-INFLAMMATORY EVALUATION OF A HYDROALCOHOLIC EXTRACT OF BLACK CURRANT LEAVES RIBES-NIGRUM" JOURNAL OF ETHNOPHARMACOLOGY, vol. 27, no. 1-2, 1989, pages 91-98, XP001025914 ISSN: 0378-8741

## Description

The present invention relates to a natural-phytotherapic composition based on hydroglyceric extracts for aerosol therapy.

More particularly, the invention relates to a natural-phytotherapic composition based on hydroglyceric extracts for treating diseases of the respiratory tract, the composition being formulated so as to be administered in spray form through apparatuses for aerosol therapy.

Inflammation of respiratory tract is indicated by symptoms like mucous hypersecretion, oedema, catarrh, cough, throat ache, difficulty in breathing, often accompanied by fever, in turn caused by bacterial, viral or mycotic infections.

Conventionally, cortisone-based, non-steroid anti-inflammatory, fluidifying and antibiotic drugs are used for treating the above inflammation.

Yet taking such drugs may have contraindications because of possible side effects, allergic reactions or intolerance of the drug by the patient.

For treating the above mentioned diseases of the respiratory tract use of natural compositions is therefore suitable, which compositions allow dispensing with taking synthetic drugs or, at least, alloe reducing the dosage so as to overcome said drawbacks.

There are known since a long time the therapeutic effects of extracts of medicinal plants such as:
- propolis, having antibacterial, antiviral, anti-inflammatory, antimycotic, immuno-stimulating, antioxidant, local anaesthetic and cicatrising effects;
- helychrysum, which has fluidifying and expectorant effects;
- ginkgo biloba, which has anti-oedema and anti-bronchoconstrictive effects;
- blackcurrant, which has anti-inflammatory, anti-reactive and diuretic effects;
- eucalyptol, which has balsamic mucolytic expectorant and antiseptic effects;
- thyme, which has bronchial antispastic effects.

Compositions containing propolis with some essential oils and/or exudates or transudates of plants for therapeutic use are also known. An example of such compositions is known from European patent application EP 0 747 057.

Yet, use of phytotherapic combinations of hydroglyceric extracts to be administered through apparatuses for aerosol therapy is not known.

Thus, it is an object of the present invention to provide a natural-phytotherapic composition for treating diseases of the respiratory tract, adapted to be administered through apparatuses for aerosol therapy.

The above and other objects of the present invention are attained through the composition based on hydroglyceric extracts, as defined in the appended claims.

Advantageously, thanks to the aerosol administration, a prolonged absorption of the active principles contained in the natural-phytotherapic composition is obtained, to perform the following pharmacological actions: fluidifying, anti-inflammatory, antibacterial, antibiotic and local anaesthetic actions.

Moreover, the composition according to the invention can be employed as an alternative to synthetic fluidifying and mucolytic drugs and can be advantageously associated with cortisone-based or antibiotic drugs, thereby allowing a reduction of the daily dosage of the latter, with clear advantages concerning tolerability and side effect reduction in patients.

Further features and advantages of the invention will become more apparent from the following description of a preferred but not exclusive embodiment thereof.

Preferably, the natural-phytotherapic composition according to the invention is obtained by mixing, in combination or in the alternative, a defined amount of:
- hydroglyceric extract of propolis;
- hydroglyceric extract of helychrysum top;
- hydroglyceric extract of ginkgo biloba leaves;
- hydroglyceric extract of blackcurrant buds;
   with eucalyptol, natural flavours (mint, menthol, anethol), tea plant essential oil, thyme essential oil, grapefruit seed extract, polysorbate 80 and sodium chloride in purified water.

The formulation of the hydroglyceric extracts according to the invention is specifically intended to obtain an effective spraying of the composition by means of the apparatus for aerosol therapy, so as to allow a beneficial absorption of the active principles contained in the composition itself.

The natural-phytotherapic composition of hydroglyceric extracts is obtained by mixing the components in the following proportions, referred to 100 g of composition:
- hydroglyceric extract of propolis 0.5 to 20 g;
- hydroglyceric extract of helychrysum top 0.5 to 10 g;
- hydroglyceric extract of ginkgo biloba leaves 0.5 to 10 g;
- hydroglyceric extract of blackcurrant buds 1 to 20 g;
- eucalyptol 0.005 g;
- natural flavours 0.005 g;
- essential oil of tea pant 0.005 g;
- thyme essential oil 0.005 g;
- grapefruit seed extract 0.020 g;
- polysorbate 80 0.20 g;
- sodium chloride (Italian Official Pharmacopoeia) 0.9 g;
- purified water: the balance to 100 g.

### Example

A particularly effective composition has been made with the following proportions, referred to 100 g of composition:
- hydroglyceric extract of propolis 2 g;
- hydroglyceric extract of helychrysum top 2 g;
- hydroglyceric extract of ginkgo biloba leaves 1 g;
- hydroglyceric extract of blackcurrant buds 5 g;
- eucalyptol 0.005 g;
- natural flavours 0.005 g;
- tea plant essential oil 0.005 g;
- thyme essential oil 0.005 g;
- grapefruit seed extract 0.020 g;
- polysorbate 80 0.20 g;
- sodium chloride (Italian Official Pharmacopoeia) 0.9 g;
- purified water 88.86 g.

The above composition has proved to be particularly effective and exhibited clear fluidifying, expectorant, anti-cough, anti-inflammatory, antibiotic and local anaesthetic actions.

## Claims

1. A natural-phytotherapic composition based on hydroglyceric extracts for aerosol therapy, **characterised in that** it comprises, referred to 100 g of composition:
- hydroglyceric extract (extractum hydroglycericum) of propolis 0.5 to 20 g;
- hydroglyceric extract of helychrysum top 0.5 to 10 g;
- hydroglyceric extract of ginkgo biloba leaves 0.5 to 10 g;
- hydroglyceric extract of blackcurrant buds 1 to 20 g;
- eucalyptol 0.005 g;
- natural flavours 0.005 g;
- tea pant essential oil 0.005 g;
- thyme essential oil 0.005 g;
- grapefruit seed extract 0.020 g;
- polysorbate 80 0.20 g;
- sodium chloride (Italian Official Pharmacopoeia) 0.9 g;
- purified water: the balance to 100 g.

2. A natural-phytotherapic composition according to claim 1, wherein said hydroglyceric extract of propolis is present in an amount of 2 g per 100 g composition.

3. A natural-phytotherapic composition according to claim 1, wherein said hydroglyceric extract of helychrysum top is present in an amount of 2 g per 100 g composition.

4. A natural-phytotherapic composition according to claim 1, wherein said hydroglyceric extract of ginkgo biloba leaves is present in an amount of 1 g per 100 g composition.

5. A natural-phytotherapic composition according to claim 1, wherein said hydroglyceric extract of blackcurrant buds is present in an amount of 5 g per 100 g composition.

## Patentansprüche

1. Naturalphytotherapeutische Mischung auf Hydroglycerol-Extrakt-Basis zur Aerosol-Therapie,
**dadurch gekennzeichnet, dass** sie, auf 100g Mischung bezogen, Folgendes enthält:
- Hydroglycerol-Auszug (extractum hydroglycericum) von Propolis 0,5 bis 20 g;
- Hydroglycerol-Auszug von Strohblumenspitzen (helichrysum) 0,5 bis 10 g;
- Hydroglycerol-Auszug von Ginko biloba Blättern 0,5 bis 10 g;
- Hydroglycerol-Auszug von Schwarzen Johannisbeerknospen (ribes nigrum) 1 bis 20 g;
- Eukalyptol 0,005 g;
- Natürliche Aromastoffe 0,005 g;
- Teepflanzenauszugsöl 0,005 g;
- Thymianauszugsöl (thymus) 0,005 g;
- Grapefruitsamen-Auszug 0,020 g;
- Polysorbat 80 0,20 g;
- Natriumchlorid (Italienisches Arzneibuch) 0,9 g;
- Destilliertes Wasser: Abgleich auf 100 g.

2. Naturalphytotherapeutische Mischung nach Anspruch 1,
worin jener Hydroglycerol-Auszug von Propolis in einer Konzentration von 2 g auf 100 g Mischung vorliegt.

3. Naturalphytotherapeutische Mischung nach Anspruch 1,
worin jener Hydroglycerol-Auszug von Strohblumenspitzen (helichrysum top) in einer Konzentration von 2 g auf 100 g Mischung vorliegt.

4. Naturalphytotherapeutische Mischung nach Anspruch 1,
worin jener Hydroglycerol-Auszug von Ginko biloba Blättern in einer Konzentration von 1 g auf 100 g Mischung vorliegt.

5. Naturalphytotherapeutische Mischung nach Anspruch 1,
worin jener Hydroglycerol-Auszug von Schwarzen Johannisbeerknospen (ribes nigrum) in einer Konzentration von 5 g auf 100 g Mischung vorliegt.

## Revendications

1. Composition phytothérapeutique naturelle à base d'extraits hydroglycériques pour thérapie par aérosols, **caractérisée en ce qu'**elle comprend, pour 100 g de composition :
- 0,5 à 20 g d'extrait hydroglycérique (*extractum hydroglycericum*) de propolis ;
- 0,5 à 10 g d'extrait hydroglycérique de têtes d'*Hélichrysum ;*
- 0,5 à 10 g d'extrait hydroglycérique de feuilles de *Ginkgo biloba ;*
- 1 à 20 g d'extrait hydroglycérique de bourgeons de cassis ;
- 0,005 g d'eucalyptol ;
- 0,005 g d'arômes naturels ;
- 0,005 g d'huile essentielle de théier ;
- 0,005 g d'huile essentielle de thym ;
- 0,020 g d'extrait de pépins de pamplemousse ;
- 0,20 g de polysorbate 80 ;
- 0,9 g de chlorure de sodium (Pharmacopée Officielle Italienne) ;
- de l'eau purifiée en quantité suffisante pour 100 g.

2. Composition phytothérapeutique naturelle selon la revendication 1, dans laquelle ledit extrait hydroglycérique de propolis est présent à raison de 2 g pour 100 g de composition.

3. Composition phytothérapeutique naturelle selon la revendication 1, dans laquelle ledit extrait hydroglycérique de têtes d'*Hélichrysum* est présent à raison de 2 g pour 100 g de composition.

4. Composition phytothérapeutique naturelle selon la revendication 1, dans laquelle ledit extrait hydroglycérique de feuilles de *Ginkgo biloba* est présent à raison de 1 g pour 100 g de composition.

5. Composition phytothérapeutique naturelle selon la revendication 1, dans laquelle ledit extrait hydroglycérique de bourgeons de cassis est présent à raison de 5 g pour 100 g de composition.
